# EUROPEAN PATENT APPLICATION

(11) **EP 1 336 405 A1**
(43) Date of publication of application: **20.08.2003**
(21) Application number: 03405088.0
(22) Date of filing: 14.02.2003
(51) Int. Cl.: A61K 9/14, A61K 31/40, A61P 3/06

(54) **Formulations of atorvastatin stabilized with alkali metal additions**

(30) Priority: 14.02.2002 IN DE01122002
(71) Applicant: RANBAXY LABORATORIES, LTD., New Delhi 110 019 (IN)
(72) Inventor: Singh, Romi Bharat, Varanasi, Uttar Pradesh 221002 (IN); Kumar, Pananchukunnath Manoj, Vikas Puri, New Delhi, Delhi 110018 (IN); Malik, Rajiv, Alaknanda, New Delhi, Delhi 110019 (IN)
(74) Representative: Cronin, Brian Harold John

(57) **Abstract**

A pharmaceutical formulation having improved bioavailability and bioequivalence includes particles of amorphous and/or crystalline atorvastatin that have a particle size (d₉₀) less than 150 µm and a mean particle size (d₅₀) of the atorvastatin particles that is between approximately 5 and 50 µm. The atorvastatin can be one or more of atorvastatin calcium, atorvastatin magnesium, atorvastatin aluminum, atorvastatin iron, and atorvastatin zinc. The formulations of atorvastatin can be stabilized by mixing atorvastatin with an alkali metal salt additive at between approximately 1.2% and less than 5% w/w of the formulation. The alkali metal salt additive may be one or more of sodium carbonate, sodium bicarbonate, sodium hydroxide, sodium silicate, disodium hydrogen orthophosphate, sodium dihydrogen phosphate, disodium hydrogen phosphate, sodium phosphate, sodium aluminate, calcium carbonate, calcium hydroxide, magnesium carbonate, magnesium hydroxide, magnesium silicate, magnesium aluminate, and aluminum magnesium hydroxide. The atorvastatin formulations can be used to treat medical conditions, including primary hypercholesterolemia, dysbetalipoproteinemia, and homozygous familial hypercholesterolemia.

## Description

### Technical Field

The technical field of the invention relates to stabilized atorvastatin, and more particularly to amorphous and crystalline atorvastatins and formulations stabilized with alkali metal salt additives and formulated with a small particle size of atorvastatin.

### Background

Atorvastatin, which is an inhibitor of the enzyme 3-hydroxy-3-methyl glutaryl coenzyme A reductase (HMG-CoA reductase), is commercially available for the treatment of primary hypercholesterolemia, dysbetalipoproteinemia and homozygous familial hypercholesterolemia. One form of atorvastatin, atorvastatin calcium, has the general formula:

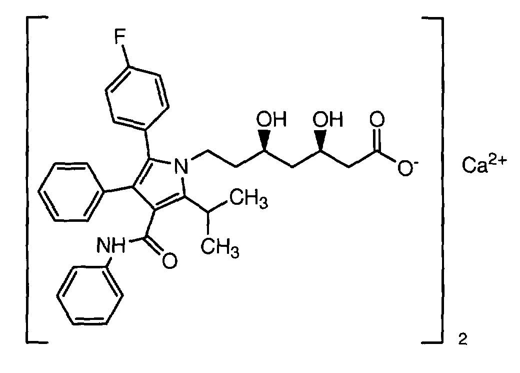

Although cholesterol is an indispensable component of all cell membranes as well as a precursor of a variety of steroid hormones and bile acids, excessively high levels of blood cholesterol and lipids increase the risk of the onset of atherosclerosis and coronary heart disease. The blood cholesterol pool is generally dependent upon dietary uptake of cholesterol and the biosynthesis of cholesterol. HMG-CoA reductase enzyme inhibitors, such as atorvastatin, bring about a reduction in the levels of blood cholesterol, especially the low-density lipoproteins, by inhibiting the synthesis of cholesterol. They are therefore excellent candidates for controlling blood cholesterol levels.

According to the U.S. Food and Drug Administration's (FDA) summary basis of approval (SBA) for Warner-Lambert's Lipitor™, atorvastatin is present in multiple amorphous and crystalline forms. Originally, atorvastatin was synthesized in the amorphous form and most of the clinical pharmacology studies were conducted on tablets prepared from this material. This form of atorvastatin was reported to be hygroscopic and unstable when exposed to oxygen. Later, a more stable crystalline form of atorvastatin was developed. Clinical studies on crystalline atorvastatin did not show any adverse implications over the amorphous form. Bioavailability studies on the tablets prepared using the crystalline form of atorvastatin, on the other hand, showed that the rate of atorvastatin absorption was significantly higher (i.e., an approximate 50% increase in Cₘₐₓ) for these tablets than for tablets prepared by using the amorphous form of atorvastatin. The extent of atorvastatin absorption as determined from the area under the plasma concentration-time curve (AUC) was, however, the same for the two formulations. Due to its better stability and faster absorption from the gastrointestinal tract, crystalline atorvastatin is being used in the marketed formulation of atorvastatin.

Despite its better stability, crystalline atorvastatin is highly susceptible to heat, moisture, a low pH environment, and light. In an acidic environment, in particular, atorvastatin degrades into corresponding lactone. It may also be or is also further destabilized on contact with excipients, such as binders, diluents, and surfactants, when formulated in the form of tablets, powders or other dosage forms.

Various attempts have been made to stabilize atorvastatin. WO 00/35425 discloses attempts to stabilize statin formulations using buffering agents capable of providing a pH in the range from 7 to 11.

U.S. Patent No. 5,686,104 and U.S. Patent No. 6,126,971 disclose oral pharmaceutical formulations of atorvastatin in which the formulation is described as being stabilized by the addition of a pharmaceutically acceptable alkaline earth metal salt. According to these patents, large amounts of alkaline earth metal salt are required to stabilize the formulation. For example, these patents provide examples in which the drug compositions contain approximately 22% of an alkaline earth metal salt used to stabilize the atorvastatin. Nonetheless, these patents claim and/or state that between 5% and 75% of the composition can be the alkaline earth metal salt. The alkaline earth metal salt is described as providing effective control of the microenvironment of the composition.

### Summary

In spite of the attempts in the prior art, described above, the inventors are not aware of successful attempts to stabilize the amorphous form of atorvastatin and improve its bioavailability profile. As described below, the inventors have surprisingly found that by reducing the particle size of amorphous atorvastatin, they have been able to increase its bioavailability and achieve the same or higher rate and extent of absorption (as measured by Cₘₐₓ and AUC, respectively) from the gastrointestinal tract as that achieved by the crystalline form of Atorvastatin marketed under the trade name Lipitor™ in the USA. The inventors also have surprisingly found that amorphous atorvastatin formulations can be stabilized by the use of very small amounts (e.g., less than 5% w/w) of an alkali metal salt additive. The inventors also have found that crystalline atorvastatin benefits from the same stabilization techniques that are applied to amorphous atorvastatin. Specifically, the stability of crystalline atorvastatin can be improved by adding very small amounts of an alkali metal salt additive.

In general, the inventors have developed the following concepts with advantageous benefit to stabilization and/or bioavailability: (1) stabilization of amorphous atorvastatin with approximately 1.2% to less than 5% concentration of alkali metal additives; (2) particle size of less than 150 microns of amorphous atorvastatin for equivalent or better bioequivalence relative to approved atorvastatin formulations; (3) particle size of less than 150 microns of amorphous atorvastatin and stabilization of amorphous atorvastatin with approximately 1.2% to less than 5% concentration of an alkali metal additive; and (4) stabilization of crystalline atorvastatin with approximately 1.2% to less than 5% concentration of an alkali metal additive.

In one general aspect, a pharmaceutical formulation includes particles of amorphous atorvastatin, the particles having a particle size (d₉₀) that is less than 150 µm.

Embodiments of the pharmaceutical formulation may include one or more of the following features. For example, the mean particle size (d₅₀) of the amorphous atorvastatin particles may be between approximately 5 and 50 µm. One or both of the rate and the extent of absorption of the amorphous atorvastatin may be equal to or greater than that obtained by a crystalline atorvastatin formulation marketed under the trade name Lipitor™. One or both of the 90% confidence interval for the area under the concentration time curve of atorvastatin (AUC₀₋ₜ) and maximum plasma concentration (Cₘₐₓ) values lies between 0.80-1.25 of the interval set by the U.S. Food and Drug Administration for a crystalline atorvastatin formulation marketed under the trade name Lipitor™. The amorphous atorvastatin may be present at between approximately 1% and 50% by weight of the formulation. The amorphous atorvastatin may be one or more of atorvastatin calcium, atorvastatin magnesium, atorvastatin aluminum, atorvastatin iron, and atorvastatin zinc.

The pharmaceutical formulation may further include one or more pharmaceutically acceptable excipients selected from the group of diluents, surfactants, antioxidants, disintegrants, binders, lubricants, glidants, and chelating agents.

The pharmaceutical formulation may further include an alkali metal salt additive. The alkali metal salt additive may be present at a concentration of between approximately 1.2% to less than 5% by weight of the formulation, at a concentration of between 2.0% and 4.8% by weight of the formulation, or at a concentration of between 4.3% and 4.4% by weight of the formulation.

The alkali metal salt additive may be one or more of sodium carbonate, sodium bicarbonate, sodium hydroxide, sodium silicate, disodium hydrogen orthophosphate, sodium dihydrogen phosphate, disodium hydrogen phosphate, sodium phosphate, and sodium aluminate. If the alkali metal salt is one of these additives, it may be present at a concentration of between approximately 1.2% and less than 5% by weight of the formulation, at a concentration of between 2.0% and 4.8% by weight of the formulation, or at a concentration of between 4.3% and 4.4% by weight of the formulation. In particular, the alkali metal salt additive in the pharmaceutical formulation may be one or both of sodium carbonate and disodium hydrogen orthophosphate.

The alkali metal salt additive also may be one or more of calcium carbonate, calcium hydroxide, magnesium carbonate, magnesium hydroxide, magnesium silicate, magnesium aluminate, and aluminum magnesium hydroxide.

The pharmaceutical formulation may include one or more of a 10 mg atorvastatin dosage unit, a 20 mg atorvastatin dosage unit, a 40 mg atorvastatin dosage unit, and an 80 mg atorvastatin dosage unit.

In another general aspect, a pharmaceutical formulation includes atorvastatin and an alkali metal salt additive. The alkali metal salt additive is present at a concentration of between approximately 1.2% and less than 5% by weight of the formulation.

Embodiments of the formulation may include one or more of the following features. For example, the alkali metal salt additive may be present at a concentration of between 2.0% and 4.8% by weight of the formulation or at a concentration of between 4.3% and 4.4% by weight of the formulation,

The alkali metal salt additive may be one or more of sodium carbonate, sodium bicarbonate, sodium hydroxide, sodium silicate, disodium hydrogen orthophosphate, sodium dihydrogen phosphate, disodium hydrogen phosphate, sodium phosphate, sodium aluminate, calcium carbonate, calcium hydroxide, magnesium carbonate, magnesium hydroxide, magnesium silicate, magnesium aluminate, and aluminum magnesium hydroxide. In particular, the alkali metal salt additive in the pharmaceutical formulation may be one or both of sodium carbonate and disodium hydrogen orthophosphate. If the alkali metal salt additive is one or more of the additives from this list, it may be present at a concentration of between approximately 1.2% and less than 5% by weight of the formulation, at a concentration of between 2.0% and 4.8% by weight of the formulation, or at a concentration of between 4.3% and 4.4% by weight of the formulation.

The atorvastatin may be in the form of particles of amorphous atorvastatin and the particles have a particle size (d₉₀) less than 150 µm. The mean particle size (d₅₀) of the amorphous atorvastatin particles may be between approximately 5 and 50 µm.

The amorphous atorvastatin may be one or more of atorvastatin calcium, atorvastatin magnesium, atorvastatin aluminum, atorvastatin iron, and atorvastatin zinc.

The pharmaceutical formulation may include one or more of a 10 mg atorvastatin dosage unit, a 20 mg atorvastatin dosage unit, a 40 mg atorvastatin dosage unit, and an 80 mg atorvastatin dosage unit. The pharmaceutical formulation may be a tablet or a capsule, and the tablet may be coated.

In another general aspect, a method of making a pharmaceutical formulation includes reducing the particle size of particles of amorphous atorvastatin, forming a mixture by mixing the reduced-size particles of amorphous atorvastatin with one or more pharmaceutical excipients, and compressing the mixture into a pharmaceutical dosage form.

Embodiments of the method of making the pharmaceutical formulation may include one or more of the following features. For example, the particle size reduction may be carried out using one or more of air jet milling, ball milling, cad milling, and multi milling. The size of the particles of amorphous atorvastatin may be reduced to have a particle size (d₉₀) that is less than 150 µm. The size of the particles of amorphous atorvastatin may be reduced to have a mean particle size (d₅₀) that is between 5 and 50 µm.

The pharmaceutical dosage form may include between 1% and 50% by weight of amorphous atorvastatin. The amorphous atorvastatin may be one or more of atorvastatin calcium, atorvastatin magnesium, atorvastatin aluminum, atorvastatin iron, and atorvastatin zinc.

The method of making the pharmaceutical formulation may further include mixing an alkali metal salt additive with the amorphous atorvastatin. The alkali metal salt additive may be mixed in at a weight percentage of between approximately 1.2% to less than 5% by weight of the formulation, or between 2.0% and 4.8% by weight of the formulation, or between 4.3% and 4.4% by weight of the formulation. The alkali metal salt additive may be one or more of sodium carbonate, sodium bicarbonate, sodium hydroxide, sodium silicate, disodium hydrogen orthophosphate, sodium dihydrogen phosphate, disodium hydrogen phosphate, sodium phosphate, and sodium aluminate. In particular, the alkali metal salt additive may be one or both of sodium carbonate and disodium hydrogen orthophosphate. The alkali metal salt additive also may be one or more of calcium carbonate, calcium hydroxide, magnesium carbonate, magnesium hydroxide, magnesium silicate, magnesium aluminate, and aluminum magnesium hydroxide.

The pharmaceutical excipients may be one or more pharmaceutically acceptable excipients such as diluents, surfactants, antioxidants, disintegrants, binders, lubricants, glidants, and chelating agents.

The pharmaceutical dosage may be one or more of a 10 mg atorvastatin dosage unit, a 20 mg atorvastatin dosage unit, a 40 mg atorvastatin dosage unit, and an 80 mg atorvastatin dosage unit. The pharmaceutical dosage may be a tablet or a capsule, and the tablet may be coated.

In another general aspect, a method of making a pharmaceutical formulation includes providing particles of amorphous atorvastatin, providing an alkali metal salt additive, mixing the particles of amorphous atorvastatin with the alkali metal salt additive, and compressing the mixture into a dosage form.

Embodiments of the method of making the pharmaceutical formulation may include one or more of the following features. For example, the alkali metal salt additive may be at a weight percentage of between approximately 1.2% and less than 5% by weight of the formulation, at between approximately 2.0% and 4.8% by weight of the formulation, or at between approximately 4.3% and 4.4% by weight of the formulation. The alkali metal salt additive may be one or more of sodium carbonate, sodium bicarbonate, sodium hydroxide, sodium silicate, disodium hydrogen orthophosphate, sodium dihydrogen phosphate, disodium hydrogen phosphate, sodium phosphate, and sodium aluminate. In particular, the alkali metal salt additive may be one or both of sodium carbonate and disodium hydrogen orthophosphate. The alkali metal salt additive also may be one or more of calcium carbonate, calcium hydroxide, magnesium carbonate, magnesium hydroxide, magnesium silicate, magnesium aluminate, and aluminum magnesium hydroxide.

The method of making the pharmaceutical formulation may further include reducing the particle size of the particles of amorphous atorvastatin in a particle size reducing operation. The particle size reducing operation may be one or more of air jet milling, ball milling, cad milling, and multi milling. The particle size reducing operation may reduce the size of the particles of amorphous atorvastatin to have a particle size (d₉₀) that is less than 150 µm. The particle size reducing operation may reduce the size of the particles of amorphous atorvastatin to have a mean particle size (d₅₀) that is between approximately 5 and 50 µm.

The dosage form may include between approximately 1% and 50% by weight of amorphous atorvastatin. The amorphous atorvastatin may be one or more of atorvastatin calcium, atorvastatin magnesium, atorvastatin aluminum, atorvastatin iron, and atorvastatin zinc.

The method of making the pharmaceutical formulation may further include mixing the amorphous atorvastatin and alkali metal salt additive with one or more pharmaceutical excipients selected from the group comprising diluents, surfactants, antioxidants, disintegrants, binders, lubricants, glidants, and chelating agents.

The dosage form may include one or more of a 10 mg atorvastatin dosage unit, a 20 mg atorvastatin dosage unit, a 40 mg atorvastatin dosage unit, and an 80 mg atorvastatin dosage unit. The dosage form may be a tablet or capsule, and the method may further include coating the tablet.

In another general aspect, a method of treatment for a medical condition includes providing an oral pharmaceutical dosage comprising a therapeutically effective dosage of atorvastatin to treat the medical condition. A majority of the atorvastatin in the pharmaceutical dosage comprises amorphous atorvastatin.

Embodiments of the method of treatment may include one or more of the following features. For example, the medical condition treated may include one or more of primary hypercholesterolemia, dysbetalipoproteinemia, and homozygous familial hypercholesterolemia.

The amorphous atorvastatin of the oral pharmaceutical dosage may be stabilized in the dosage by an alkali metal salt additive. The alkali metal salt additive may be present at between approximately 1.2% and less than 5% by weight of the formulation. The amorphous atorvastatin may be in the form of particles having a particle size (d₉₀) that is less than 150 µm. The amorphous atorvastatin may be in the form of particles having a mean particle size (d₅₀) that is between approximately 5 and 50 µm.

The oral pharmaceutical dosage may include one or more of a 10 mg atorvastatin dosage unit, a 20 mg atorvastatin dosage unit, a 40 mg atorvastatin dosage unit, and an 80 mg atorvastatin dosage unit.

In another general aspect, a method of stabilizing a pharmaceutical formulation of amorphous atorvastatin includes mixing amorphous atorvastatin with an alkali metal salt additive, the alkali metal salt additive being present at between approximately 1.2% and less than 5% by weight of the formulation.

Another general aspect relates to a method of improving bioavailability of a pharmaceutical formulation that includes atorvastatin in comparison to a pharmaceutical formulation that includes atorvastatin marketed under the trade name Lipitor™. The method of improving bioavailability includes reducing the particle size of the amorphous atorvastatin such that the particles have a particle size (d₉₀) that is less than 150 µm and a mean particle size (d₅₀) that is between approximately 5 and 50 µm.

In another general aspect, a method of processing amorphous atorvastatin to reduce the particle size of the amorphous atorvastatin include milling the amorphous atorvastatin particles using one or more of an air jet milling, ball milling, cad milling, and multi milling operation to reduce the particle size of the amorphous atorvastatin such that the particles have a particle size (d₉₀) that is less than 150 µm and a mean particle size (d₅₀) that is between approximately 5 and 50 µm.

In another general aspect, a pharmaceutical formulation includes crystalline atorvastatin and an alkali metal salt additive, the alkali metal salt additive being present at between approximately 1.2% and less than 5% by weight of the formulation.

Embodiments of the pharmaceutical formulation may include one or more of the following features. For example, the alkali metal salt additive may be present at between approximately 2.0% and 4.8% by weight of the formulation or at between approximately 4.3% and 4.4% by weight of the formulation. The alkali metal salt additive may be one or more of sodium carbonate, sodium bicarbonate, sodium hydroxide, sodium silicate, disodium hydrogen orthophosphate, sodium dihydrogen phosphate, disodium hydrogen phosphate, sodium phosphate, sodium aluminate, calcium carbonate, calcium hydroxide, magnesium carbonate, magnesium hydroxide, magnesium silicate, magnesium aluminate, and aluminum magnesium hydroxide.

The details of one or more embodiments of the inventions are set forth in the description below. Other features, objects, and advantages of the inventions will be apparent from the description and the claims.

### Description

As used herein the term "atorvastatin" refers to atorvastatin calcium, atorvastatin magnesium, atorvastatin aluminum, atorvastatin iron, atorvastatin zinc, and other suitable salts of atorvastatin.

The term "stable pharmaceutical formulation" is used herein to mean that after storage for three months at 40° C and 75% relative humidity, no more than about 10%, in particular no more than about 5% and more particularly, no more than about 2% by weight of the active component initially present in the composition degrades into the corresponding lactone.

The inventors have developed advantageous formulations of atorvastatin for treatment of medical conditions, including primary hypercholesterolemia, dysbetalipoproteinemia and homozygous familial hypercholesterolemia. Atorvastatin is present in the formulations at a dosage of between about 1% to about 50% by weight of the composition.

In preparing the formulations, amorphous atorvastatin particles were reduced in size in a particle size reduction step using conventional milling techniques, such as air jet milling, ball milling, cad milling, multi milling and other suitable size reduction techniques. The particle size of the atorvastatin was reduced to a mean particle size d₉₀ of less than approximately 150 µm, and more particularly to a mean particle size of between approximately 5 µm and 50 µm. The size of the particles was analyzed using a conventional particle size analyzer (e.g., a Malvern Master Sizer), although any conventional particle size analyzer is suitable for particle size analysis.

The stabilizing alkali metal salt additive is selected from amongst one or more of sodium carbonate, sodium hydroxide, sodium silicate, disodium hydrogen orthophosphate, sodium aluminate and other suitable alkali metal salts. In particular, the stabilizing alkali metal salt additive may be selected from amongst sodium carbonate and disodium hydrogen orthophosphate, although the other alkali metal salt additives may also be selected. The alkali metal salt additive is present at a concentration of between approximately 1.2 % to less than about 5% by weight of the composition. In particular, advantageous stabilization has been observed when the alkali metal salt additive is present at between approximately 2% and 4.8% by weight, and more particularly at between approximately 4.3% and 4.4% by weight. Other suitable alkali metal salt additives in addition to those above include one or more of calcium carbonate, calcium hydroxide, magnesium carbonate, magnesium hydroxide, magnesium silicate, magnesium aluminate, and aluminum magnesium hydroxide.

The formulations including one or both of the small particle sized atorvastatin and the alkali metal salt additive may further contain other pharmaceutically acceptable excipients, such as binders, diluents, disintegrants, surfactants, lubricants, antioxidants, and chelating agents. The formulations can be, for example, compressed into tablets and then optionally coated, or formed into capsules.

The binders may be selected from amongst one or more of those binders known in the art. Examples of suitable binders include, but are not limited to, starch, polyvinyl pyrrolidone, hydroxypropyl cellulose, hydroxypropyl methylcellulose, and carboxymethylcellulose.

The diluents may be selected from amongst one or more of those diluents known in the art. Examples of suitable diluents include, but are not limited to, lactose, microcrystalline cellulose, corn starch, sucrose, and silicic anhydride. The diluent may be present from about 30% to 75% by weight of the formulation.

The disintegrant may be selected from amongst one or more of those suitable disintegrants known in the art. Examples of suitable disintegrants include, but are not limited to, croscarmellose sodium and starch.

The surfactants may be selected from amongst one or more of those suitable surfactants known in the art. Examples of suitable surfactants include, but are not limited to, polysorbate 80, polyoxyethylene sorbitan, polyoxyethylene-polyoxypropylene copolymer, and sodium lauryl sulphate.

The lubricants may be selected from amongst one or more of those suitable lubricants known in the art. Examples of suitable lubricants include, but are not limited to, magnesium stearate, stearic acid, palmitic acid, talc, and aerosil.

The glidants may be selected from amongst one or more of those suitable glidants known in the art. An example of a suitable, pharmaceutically acceptable glidant includes colloidal silicon dioxide.

The pharmaceutically acceptable antioxidants may be selected from amongst one or more of those suitable antioxidants known in the art. Examples of suitable pharmaceutically acceptable antioxidants include, but are not limited to, butylated hydroxyanisole (BHA), sodium ascorbate, butylated hydroxytoluene (BHT), sodium sulfite, citric acid, malic acid, and ascorbic acid.

The chelating agents may be selected from amongst one or more of those suitable chelating agents known in the art. Examples of suitable chelating agents include, but are not limited to disodium edetate (EDTA). The chelating agents are present at a concentration of up to approximately 5% by weight of the formulations.

The coating may be selected from amongst one or more of those suitable coating materials known in the art. For example, the coating material can be Opadry or opadry AMB (aqueous moisture barrier).

The following examples further exemplify the formulations as applied to tablets that contain 80 mg of amorphous and/or crystalline atorvastatin and are not intended to limit the scope of the invention.

### Example 1

The tablets of Example 1 were formulated with milled amorphous atorvastatin and with an alkali metal salt. The amorphous atorvastatin was milled to reduce its mean particle size d₅₀ to approximately 5-20 µm and d₉₀ to approximately 15-35 µm. Polysorbate 80 (12 mg/tablet), butylated hydroxy anisole (0.12 mg/tablet), and butylated hydroxy toluene (0.12 mg/tablet) were dissolved in isopropyl alcohol, and applied on to lactose. The lactose was dried at 40-45°C in a fluidized bed dryer. Amorphous atorvastatin (80 mg/tablet), microcrystalline cellulose (300 mg/tablet) and lactose (665.5 mg/tablet) were mixed. Following the mixing, the dry binder, hydroxypropyl cellulose-L, (24 mg/tablet) and disintegrant, croscarmellose sodium, (72 mg/tablet) were added to the mixture. Following this addition, an alkali metal salt, disodium hydrogen orthophosphate (3.5 mg/tablet), chelating agent, EDTA (1 mg/tablet), and glidant, colloidal silicon dioxide (24 mg/tablet) were added. Next, the mixture was lubricated with magnesium stearate (12 mg/tablet) and compressed into tablets. The tablets then were coated with Opadry AMB. The values given above are per tablet and can be adjusted appropriately to provide the desired batch size.

### Example 2

The tablets of Example 2 were formulated with milled amorphous atorvastatin and an alkali metal salt. The amorphous atorvastatin was milled to reduce its mean particle size d₅₀ to approximately 20-50 µm and d₉₀ to approximately 80-100 µm. Butylated hydroxy anisole (0.12 mg/tablet) and butylated hydroxy toluene (0.12 mg/tablet) were dissolved in isopropyl alcohol and applied on to lactose under high shear mixing. The lactose was dried at 40-45°C in a fluidized bed dryer. Amorphous atorvastatin (80 mg/tablet), microcrystalline cellulose (300 mg/tablet) and lactose (628 mg/tablet) were mixed. Following the mixing, the dry binder, hydroxypropyl cellulose-L, (24 mg/tablet) and disintegrant, croscarmellose sodium, (72 mg/tablet) were added to the mixture. Following this addition, an alkali metal salt, sodium carbonate (52 mg/tablet), surfactant, sodium lauryl sulphate (2 mg/tablet), and colloidal silicon dioxide (24 mg/tablet) were added. Next, the mixture was lubricated with magnesium stearate (12 mg/tablet) and compressed into tablets. The tablets then were coated with Opadry AMB. The values given above are per tablet and can be adjusted appropriately to provide the desired batch size.

### Example 3

The tablets of Example 2 were formulated with non-milled amorphous atorvastatin, but without an alkali metal salt. The amorphous atorvastatin had a d₉₀ of approximately 129 µm and a d₅₀ of approximately 44µm. Amorphous atorvastatin (80 mg/tablet), microcrystalline cellulose (300 mg/tablet), and lactose (680 mg/tablet) were mixed. Following the mixing, the dry binder, hydroxypropyl cellulose-L (24 mg/tablet), and disintegrant, croscarmellose sodium (72 mg/tablet), were added to the mixture. Following this addition, surfactant, sodium lauryl sulphate (2 mg/tablet), and colloidal silicon dioxide (24 mg/tablet) were added. Next, the mixture was lubricated with magnesium stearate (12 mg/tablet) and compressed into tablets. The tablets then were coated with Opadry AMB. The values given above are per tablet and can be adjusted appropriately to provide the desired batch size.

Although the above examples were specific to atorvastatin calcium, other forms of atorvastatin can be used. For example, the tablets of Example 4 and 5 include crystalline atorvastatin magnesium in place of atorvastatin calcium, although a mixture of the two also can be used.

### Example 4

The tablets of Example 4 were formulated with non-milled atorvastatin magnesium crystalline and an alkali metal salt. The atorvastatin had a d₉₀ of approximately 237 µm and a d₅₀ of approximately 98µm. Crystalline atorvastatin magnesium (80 mg/tablet), microcrystalline cellulose (300 mg/tablet), and lactose (624 mg/tablet) were mixed. Following the mixing, the dry binder, hydroxypropyl cellulose-L, (24 mg/tablet) and disintegrant, croscarmellose sodium, (72 mg/tablet) were added to the mixture. Following this addition, an alkali metal salt, sodium carbonate (52 mg/tablet), surfactant, sodium lauryl sulphate (2 mg/tablet), and colloidal silicon dioxide (24 mg/tablet) were added. Next, the mixture was lubricated with magnesium stearate (12 mg/tablet) and compressed into tablets. The tablets then were coated with Opadry AMB. The values given above are per tablet and can be adjusted appropriately to provide the desired batch size.

### Example 5

In the first processing step, atorvastatin magnesium (80 mg/tablet) was mixed with colloidal silicon dioxide (24.0 mg/tablet) and sodium carbonate anhydrous (26.0 mg/tablet) in a low shear mixer. In the second step, the antioxidants, butylated hydroxyl anisole (0.12 mg/tablet) and butylated hydroxyl toluene (0.12 mg/tablet) were dissolved in Isopropyl Alcohol (quantities sufficient) and applied on to the anhydrous lactose in a high shear mixer. The bulk then was dried in a fluidized bed dryer at 40°C. In the third step, croscarmellose sodium (72.0 mg/tablet), hydroxyl propyl cellulose-L (24.0 mg/tablet), sodium lauryl sulphate (2.0 mg/tablet) were mixed with microcrystalline cellulose (300.0 mg/tablet), added to the bulk of the first step, and mixed in a low shear blender. In the fourth step, the mixed bulk of the third step then was added to the bulk of the second step and mixed in a low shear blender. In the fifth step, magnesium stearate (12.0 mg/tablet) was added to the bulk of the fourth step and lubricated in a low shear blender. In the sixth step, the blend from the fifth step was compressed using suitable tooling. In the seventh step, the tablets were coated with Opadry AMB.

In general, the process described above to make the tablets includes the following steps: (1) mixing atorvastatin, either crystalline and/or amorphous as such or after milling to reduce its mean particle size (d₅₀ and d₉₀), microcrystalline cellulose and lactose on to which the antioxidants are applied after dissolving in a suitable solvent; (2) adding a dry binder (e.g., hydroxypropyl cellulose-L) and disintegrant (e.g., croscarmellose sodium) to the mixture; (3) adding one or more alkali metal salts, EDTA, surfactant and glidant (e.g., colloidal silicon dioxide); (4) lubricating the mixture with magnesium stearate; and (5) compressing the lubricated mixture into tablets. An optional sixth step is coating the tablets with a coating material.

The composition of the tablets of Examples 1-5, prepared using the milled and non-milled atorvastatin with and without an alkali metal salt additive, are listed in Table 1.

**Table 1.**

| **Compositions of Tablets of Examples 1-5** | | | | | |
|---|---|---|---|---|---|
| **Ingredient** | **Example Formulation (mg per tablet)** | | | | |
| | **1** | **2** | **3** | **4** | **5** |
| Atorvastatin | 80 (Ca) | 80 (Ca) | 80* (Ca) | 80* (Mg) | 80* (Mg) |
| Lactose** | 665.5 | 628 | 680 | 624 | 651.01 |
| Microcrystalline cellulose | 300 | 300 | 300 | 300 | 300 |
| Colloidal silicon dioxide | 24 | 24 | 24 | 24 | 24 |
| Hydroxypropyl cellulose | 24 | 24 | 24 | 24 | 24 |
| Croscarmellose Sodium | 72 | 72 | 72 | 72 | 72 |
| Polysorbate 80 | 12 | -- | -- | -- | -- |
| Isopropyl alcohol | q.s. | q.s. | q.s. | q.s. | q.s. |
| Disodium hydrogen orthophosphate | 3.5 | -- | -- | -- | -- |
| Edetate disodium (EDTA) | 1.0 | -- | -- | -- | -- |
| Sodium carbonate | -- | 52 | -- | 52 | 26.0 |
| Sodium lauryl sulphate | -- | 2 | 2 | 2 | 2 |
| Magnesium Stearate | 12 | 12 | 12 | 12 | 12 |
| Butylated hydroxy anisole | 0.12 | 0.12 | 0.12 | 0.12 | 0.12 |
| Butylated hydroxy toluene | 0.12 | 0.12 | 0.12 | 0.12 | 0.12 |

| | | | | | |
|---|---|---|---|---|---|
| *Non-milled atorvastatin magnesium or calcium | | | | | |
| **Input to be adjusted based on potency and moisture correction of the atorvastatin to maintain constant tablet weight. | | | | | |

To analyze for stability, the tablets formulated above were subjected to accelerated stability testing at 40°C and 75% relative humidity (RH) for three months. As indicated in Table 2, those tablets containing an alkali metal salt additive (i.e., Example Tablets 1, 2, and 4) exhibited very good stability characteristics over the period tested.

**Table 2.**

| **Accelerated Stability Testing of Tablets of Examples 1-4** | | | | |
|---|---|---|---|---|
| **Test Period** (Testing at 40°C and 75% Relative Humidity) | **Assay of atorvastatin (% of label claim)** | | | |
| | Example 1 | Example 2 | Example 3 | Example 4 |
| Initial | 101.2 | 99.78 | 100.23 | 101.68 |
| One Month | 100.5 | 99.16 | 97.17 | 101.27 |
| Two Months | 100.09 | 99.28 | 95.52 | 100.98 |
| Three Months | 99.87 | 99.09 | 94.19 | 100.34 |

To obtain a dissolution profile, the tablets prepared according to the examples above were subjected to dissolution testing in 0.5 M phosphate buffer, pH 6.8 at 75 revolutions per minute using USP apparatus-II. The results of the dissolution testing are provided in Table 3.

**Table 3.**

| **Dissolution Profile of Tablets of Examples 1-4** | | | | |
|---|---|---|---|---|
| **Time (minutes)** | **(%) release of atorvastatin** | | | |
| | **Example 1** | **Example 2** | **Example-3** | **Example-4** |
| 10 | 93 | 60 | 91 | 45 |
| 20 | 102 | 82 | 99 | 67 |
| 30 | 102 | 91 | 100 | 84 |
| 45 | 102 | 100 | 101 | 97 |

The bioavailability of the tablets of Examples 1 and 2 was measured in a randomized three treatment, three period, three sequence single dose, crossover comparative bioavailability study against Lipitor™ 80 mg tablets manufactured by Warner-Lambert Ltd. The bioavailability studies were conducted in healthy, adult, male human subjects under fasting conditions. The results of the bioavailability studies are provided below in Table 4. These results are in the form of ratios of the test formulation (i.e., Example 1 or 2) to the reference formulation (i.e., Lipitor™).

**Table 4.**

| **Comparative Bioavailability Data of Examples 1 and 2** | | | |
|---|---|---|---|
| **Ratio of Test Formulation to Reference Formulation** | **AUC (0-infinity)** | **AUC (0-t)** | **C**_{**max**} |
| Example 1 | 134.5 | 135.5 | 143.7 |
| Example 2) | 111.6 | 110.8 | 108.8 |

As can be seen from the AUC and Cₘₐₓ values given above the formulation made in accordance with Example 2 comprising amorphous atorvastatin having a mean particle size (d₉₀) of around 100 µm was bioequivalent to the commercially available crystalline formulation of atorvastatin sold under the trade name Lipitor™. Both the Cₘₐₓ and the AUC values of this formulation fall within the 0.8-1.25 intervals mandated by the U.S. Food and Drug Administration for bioequivalence. The formulation made in accordance with Example 1 showed a higher bioavailability than the marketed crystalline formulation of atorvastatin sold under the trade name Lipitor™. The formulation of Example 1, while not falling within the 0.8-1.25 interval, demonstrates the feasibility of providing advantageous improvements in bioavailability using a reduced particle size of atorvastatin. For example, to obtain the same bioavailability as the commercially available atorvastatin, less atorvastatin can be used when a reduced particle size atorvastatin is formulated as the dosage unit. In conclusion, the inventors have been able to develop an amorphous atorvastatin formulation, which is stable but also shows similar or even higher bioavailability than Lipitor™ made from crystalline atorvastatin.

While several particular formulations have been described above, it will be apparent that various modifications and combinations of the formulations detailed in the text can be made without departing from the spirit and scope of the invention. For example, additional exemplary tablet formulations are contemplated to use the reduced particle size atorvastatin described above and the low amount of alkali metal salt additive. Specifically, reduced particle size atorvastatin formulations can be produced that have 1.04 % of the alkali metal salt, 2.08 % of the alkali metal salt, 3.13% of the alkali metal salt, and 4.3% of the alkali metal salt, as disclosed in Table 5.

**Table 5.**

| **Compositions of Tablets of Examples 5-9** | | | | |
|---|---|---|---|---|
| **Ingredient** | **mg per tablet** | | | |
| | **Example 6 (3.13% alkali metal salt)** | **Example 7 (1.04% alkali metal salt)** | **Example 8 (2.08% alkali metal salt)** | **Example 9 (4.3% alkali metal salt)** |
| Atorvastatin (amorphous) | 80 | 80 | 80 | 80 |
| Lactose | 642.5 | 667 | 655 | 628 |
| Microcrystalline cellulose | 300 | 300 | 300 | 300 |
| Colloidal silicon dioxide | 24 | 24 | 24 | 24 |
| Hydroxypropyl cellulose-L | 24 | 24 | 24 | 24 |
| Croscarmellose Sodium | 72 | 72 | 72 | 72 |
| Sodium Lauryl Sulphate | 2 | 2 | 2 | 2 |
| Butylated Hydroxy Anisole | 0.12 | 0.12 | 0.12 | 0.12 |
| Butylated Hydroxy Toluene | 0.12 | 0.12 | 0.12 | 0.12 |
| Isopropyl alcohol | q.s. | q.s. | q.s. | q.s. |
| Sodium carbonate | 37.5 | 12.5 | 25 | 52 |
| Magnesium Stearate | 12 | 12 | 12 | 12 |

Moreover, it is contemplated that the atorvastatin can be one or more of atorvastatin calcium, atorvastatin magnesium, atorvastatin aluminum, atorvastatin iron, atorvastatin zinc, and other suitable salts of atorvastatin. In addition, the atorvastatin pharmaceutical formulation can be in a tablet, capsule, or other suitable dosage form. The tablet dosage form may be coated or uncoated. It can have a nonfunctional coating to improve the aesthetic appeal or a functional coating to protect it from atmospheric moisture. The dosage forms and/or pharmaceutical formulations may include one or more of a 10 mg atorvastatin dosage unit, a 20 mg atorvastatin dosage unit, a 40 mg atorvastatin dosage unit, and an 80 mg atorvastatin dosage unit.

The utility of the atorvastatin formulations herein include the treatment of the following medical conditions: (1) as an adjunctive therapy to diet for the treatment of patients with elevated serum triglyceride levels (Frederickson Type IV); (2) for use by patients with primary dysbetalipoproteinemia (Frederickson Type III) who do not respond adequately to diet; (3) for the treatment of heterozygous familial hypercholesterolemia in adolescent boys and post-menarchal girls, ages 10 to 17 (10 to 20 mg once daily); (4) for use in increasing HDL-C in patients with primary hypercholesterolemia (heterozygous familial and nonfamilial) and mixed dyslipidemia (Frederickson Types IIa and IIb); (5) for use as an adjunct to diet to reduce elevated total-C, LDL-C, apo B, and TG levels and to increase HDL-C in patients with primary hypercholesterolemia (heterozygous familial and non-familial) and mixed dyslipidemia (Frederickson Types IIa and IIb); (6) for use in treating primary hypercholesterolemia; (7) for use in treating dysbetalipoproteinemia; and (8) for use in treating homozygous familial hypercholesterolemia. The treatment of these medical conditions includes providing an oral pharmaceutical dosage that includes a therapeutically effective dosage of atorvastatin to treat the medical condition, a majority of the atorvastatin in the pharmaceutical dosage being amorphous atorvastatin.

It also is contemplated that the methods and values provided above can be used to improve the bioavailability of a pharmaceutical formulation that includes atorvastatin in comparison to a pharmaceutical formulation that includes atorvastatin marketed under the trade name Lipitor™. Improving the bioavailability includes reducing the particle size of the amorphous atorvastatin such that the particles have a particle size (d₉₀) that is less than 150 µm and a mean particle size (d₅₀) that is between approximately 5 and 50 µm.

It also is contemplated that amorphous atorvastatin can be processed by a pharmaceutical manufacturer to reduce the particle size of the amorphous atorvastatin by milling the amorphous atorvastatin particles using one or more of an air jet milling, ball milling, cad milling, and multi milling operation. The operation reduces the particle size of the amorphous atorvastatin such that the particles have a particle size (d₉₀) that is less than 150 µm and a mean particle size (d₅₀) that is between approximately 5 and 50 µm. The advantages found in reducing the particle size of amorphous atorvastatin can be beneficial to a pharmaceutical manufacturer that produces amorphous atorvastatin, reduces the particle size in a milling operation, and provides the reduced-particle size amorphous atorvastatin to other pharmaceutical companies for production of dosage forms with improved bioavailability and bioequivalence.

The advantages found in mixing amounts of less than 5% of an alkali metal salt additive, and more particularly between 1.2% and less than 5% of an alkali metal salt additive, with the amorphous atorvastatin can be beneficial in producing stabilized amorphous atorvastatin for use in either producing stabilized amorphous atorvastatin pharmaceutical compositions or providing stabilized amorphous atorvastatin compositions to pharmaceutical companies for use in producing stabilized amorphous atorvastatin pharmaceutical dosages and products.

It also is contemplated that any single feature or any combination of optional features of the variations described herein may be specifically excluded from the claimed invention and be so described as a negative limitation. Accordingly, it is not intended that the invention be limited, except as by the claims.

While the present invention has been described in terms of its specific embodiments, certain modifications and equivalents will be apparent to those skilled in the art and are intended to be included within the scope of the present invention.

## Claims

1. A pharmaceutical formulation comprising particles of amorphous atorvastatin, wherein the particles have a particle size (d₉₀) less than 150 µm.

2. The pharmaceutical formulation of claim 1, wherein the mean particle size (d₅₀) of the amorphous atorvastatin particles is between approximately 5 and 50 µm.

3. The pharmaceutical formulation of claim 1, wherein one or both of the rate and the extent of absorption of the amorphous atorvastatin is equal to or greater than that obtained by a crystalline atorvastatin formulation marketed under the trade name Lipitor™.

4. The pharmaceutical formulation of claim 1, wherein one or both of the 90% confidence interval for the area under the concentration time curve of atorvastatin (AUC₀₋ₜ) and maximum plasma concentration (Cₘₐₓ) values lies between 0.80-1.25 of the interval set by the U.S. Food and Drug Administration for a crystalline atorvastatin formulation marketed under the trade name Lipitor™.

5. The pharmaceutical formulation of claim 1, wherein the amorphous atorvastatin comprises between approximately 1% and 50% by weight of the formulation.

6. The pharmaceutical formulation of claim 1, wherein the amorphous atorvastatin comprises one or more of atorvastatin calcium, atorvastatin magnesium, atorvastatin aluminum, atorvastatin iron, and atorvastatin zinc.

7. The pharmaceutical formulation of claim 1, further comprising one or more pharmaceutically acceptable excipients selected from the group comprising diluents, surfactants, antioxidants, disintegrants, binders, lubricants, glidants and chelating agents.

8. The pharmaceutical formulation of claim 1, further comprising an alkali metal salt additive.

9. The pharmaceutical formulation of claim 8, wherein the alkali metal salt additive is present at a concentration of between approximately 1.2% to less than 5% by weight of the formulation.

10. The pharmaceutical formulation of claim 8, wherein the alkali metal salt additive is present at a concentration of between 2.0% and 4.8% by weight of the formulation.

11. The pharmaceutical formulation of claim 8, wherein the alkali metal salt additive is present at a concentration of between 4.3% and 4.4% by weight of the formulation.

12. The pharmaceutical formulation of claim 8, wherein the alkali metal salt additive comprises one or more of sodium carbonate, sodium bicarbonate, sodium hydroxide, sodium silicate, disodium hydrogen orthophosphate, sodium dihydrogen phosphate, disodium hydrogen phosphate, sodium phosphate, and sodium aluminate.

13. The pharmaceutical formulation of claim 12, wherein the alkali metal salt additive is present at a concentration of between approximately 1.2% and less than 5% by weight of the formulation.

14. The pharmaceutical formulation of claim 12, wherein the alkali metal salt additive is present at a concentration of between 2.0% and 4.8% by weight of the formulation.

15. The pharmaceutical formulation of claim 12, wherein the alkali metal salt additive is present at a concentration of between 4.3% and 4.4% by weight of the formulation.

16. The pharmaceutical formulation of claim 8, wherein the alkali metal salt additive comprises sodium carbonate.

17. The pharmaceutical formulation of claim 8, wherein the alkali metal salt additive comprises disodium hydrogen orthophosphate.

18. The pharmaceutical formulation of claim 8, wherein the alkali metal salt additive comprises one or more of calcium carbonate, calcium hydroxide, magnesium carbonate, magnesium hydroxide, magnesium silicate, magnesium aluminate, and aluminum magnesium hydroxide.

19. The pharmaceutical formulation of claim 1, wherein the pharmaceutical formulation comprises one or more of a 10 mg atorvastatin dosage unit, a 20 mg atorvastatin dosage unit, a 40 mg atorvastatin dosage unit, and an 80 mg atorvastatin dosage unit.

20. A pharmaceutical formulation comprising atorvastatin and an alkali metal salt additive, the alkali metal salt additive being present at a concentration of between approximately 1.2% and less than 5% by weight of the formulation.

21. The pharmaceutical formulation of claim 20, wherein the alkali metal salt additive is present at a concentration of between 2.0% and 4.8% by weight of the formulation.

22. The pharmaceutical formulation of claim 20, wherein the alkali metal salt additive is present at a concentration of between 4.3% and 4.4% by weight of the formulation.

23. The pharmaceutical formulation of claim 20, wherein the alkali metal salt additive comprises one or more of sodium carbonate, sodium bicarbonate, sodium hydroxide, sodium silicate, disodium hydrogen orthophosphate, sodium dihydrogen phosphate, disodium hydrogen phosphate, sodium phosphate, sodium aluminate, calcium carbonate, calcium hydroxide, magnesium carbonate, magnesium hydroxide, magnesium silicate, magnesium aluminate, and aluminum magnesium hydroxide.

24. The pharmaceutical formulation of claim 23, wherein the alkali metal salt additive is present at a concentration of between approximately 1.2% and less than 5% by weight of the formulation.

25. The pharmaceutical formulation of claim 23, wherein the alkali metal salt additive is present at a concentration of between 2.0% and 4.8% by weight of the formulation.

26. The pharmaceutical formulation of claim 23, wherein the alkali metal salt additive is present at a concentration of between 4.3% and 4.4% by weight of the formulation.

27. The pharmaceutical formulation of claim 20, wherein the alkali metal salt additive comprises sodium carbonate.

28. The pharmaceutical formulation of claim 20, wherein the alkali metal salt additive comprises disodium hydrogen orthophosphate.

29. The pharmaceutical formulation of claim 20, wherein the atorvastatin comprises particles of amorphous atorvastatin and the particles have a particle size (d₉₀) less than 150 µm.

30. The pharmaceutical formulation of claim 20, wherein the atorvastatin comprises particles of amorphous atorvastatin and the mean particle size (d₅₀) of the amorphous atorvastatin particles is between approximately 5 and 50 µm.

31. The pharmaceutical formulation of claim 20, wherein the amorphous atorvastatin comprises one or more of atorvastatin calcium, atorvastatin magnesium, atorvastatin aluminum, atorvastatin iron, and atorvastatin zinc.

32. The pharmaceutical formulation of claim 20, wherein the pharmaceutical formulation comprises one or more of a 10 mg atorvastatin dosage unit, a 20 mg atorvastatin dosage unit, a 40 mg atorvastatin dosage unit, and an 80 mg atorvastatin dosage unit.

33. The pharmaceutical formulation of claim 20, wherein the pharmaceutical formulation comprises a tablet.

34. The pharmaceutical formulation of claim 33, further comprising a coating on the tablet.

35. The pharmaceutical formulation of claim 20, wherein the pharmaceutical comprises a capsule.

36. A method of making a pharmaceutical formulation, the method comprising:
reducing the particle size of particles of amorphous atorvastatin;
forming a mixture by mixing the reduced-size particles of amorphous atorvastatin with one or more pharmaceutical excipients; and
compressing the mixture into a pharmaceutical dosage form.

37. The method of claim 36, wherein the particle size reduction is carried out using one or more of air jet milling, ball milling, cad milling, and multi milling.

38. The method of claim 36, wherein the size of the particles of amorphous atorvastatin is reduced to have a particle size (d₉₀) that is less than 150 µm.

39. The method of claim 36, wherein the size of the particles of amorphous atorvastatin is reduced to have a mean particle size (d₅₀) of the amorphous atorvastatin particles that is between 5 and 50 µm.

40. The method of claim 36, wherein the pharmaceutical dosage form comprises between 1% and 50% by weight of amorphous atorvastatin.

41. The method of claim 36, wherein the amorphous atorvastatin comprises one or more of atorvastatin calcium, atorvastatin magnesium, atorvastatin aluminum, atorvastatin iron, and atorvastatin zinc.

42. The method of claim 36, further comprising mixing an alkali metal salt additive with the amorphous atorvastatin.

43. The method of claim 42, wherein the alkali metal salt additive is mixed in at a weight percentage of between approximately 1.2% to less than 5% by weight of the formulation.

44. The method of claim 43, wherein the alkali metal salt additive is present at between 2.0% and 4.8% by weight of the formulation.

45. The method of claim 43, wherein the alkali metal salt additive is present at between 4.3% and 4.4% by weight of the formulation

46. The method of claim 42, wherein the alkali metal salt additive comprises one or more of sodium carbonate, sodium bicarbonate, sodium hydroxide, sodium silicate, disodium hydrogen orthophosphate, sodium dihydrogen phosphate, disodium hydrogen phosphate, sodium phosphate, and sodium aluminate.

47. The method of claim 42, wherein the alkali metal salt additive comprises sodium carbonate.

48. The method of claim 42, wherein the alkali metal salt additive comprises disodium hydrogen orthophosphate.

49. The method of claim 42, wherein the alkali metal salt additive comprises one or more of calcium carbonate, calcium hydroxide, magnesium carbonate, magnesium hydroxide, magnesium silicate, magnesium aluminate, and aluminum magnesium hydroxide.

50. The method of claim 36, wherein the one or more pharmaceutical excipients comprises one or more pharmaceutically acceptable excipients selected from the group comprising diluents, surfactants, antioxidants, disintegrants, binders, lubricants, glidants, and chelating agents.

51. The method of claim 36, wherein the pharmaceutical dosage comprises a tablet.

52. The method of claim 51, further comprising coating the tablet.

53. The method of claim 36, wherein the pharmaceutical dosage comprises a capsule.

54. The method of claim 36, wherein the pharmaceutical dosage comprises one or more of a 10 mg atorvastatin dosage unit, a 20 mg atorvastatin dosage unit, a 40 mg atorvastatin dosage unit, and an 80 mg atorvastatin dosage unit.

55. A method of making a pharmaceutical formulation, the method comprising:
providing particles of amorphous atorvastatin;
providing an alkali metal salt additive;
mixing the particles of amorphous atorvastatin with the alkali metal salt additive; and
compressing the mixture into a dosage form.

56. The method of claim 55, wherein the alkali metal salt additive is provided at a weight percentage of between approximately 1.2% and less than 5% by weight of the formulation.

57. The method of claim 56, wherein the alkali metal salt additive is present at between approximately 2.0% and 4.8% by weight of the formulation.

58. The method of claim 56, wherein the alkali metal salt additive is present at between approximately 4.3% and 4.4% by weight of the formulation.

59. The method of claim 56, wherein the alkali metal salt additive comprises one or more of sodium carbonate, sodium bicarbonate, sodium hydroxide, sodium silicate, disodium hydrogen orthophosphate, sodium dihydrogen phosphate, disodium hydrogen phosphate, sodium phosphate, and sodium aluminate.

60. The method of claim 56, wherein the alkali metal salt additive comprises sodium carbonate.

61. The method of claim 56, wherein the alkali metal salt additive comprises disodium hydrogen orthophosphate.

62. The method of claim 56, wherein the alkali metal salt additive comprises one or more of calcium carbonate, calcium hydroxide, magnesium carbonate, magnesium hydroxide, magnesium silicate, magnesium aluminate, and aluminum magnesium hydroxide.

63. The method of claim 55, further comprising reducing the particle size of the particles of amorphous atorvastatin in a particle size reducing operation.

64. The method of claim 63, wherein the particle size reducing operation comprises one or more of air jet milling, ball milling, cad milling, and multi milling.

65. The method of claim 63, wherein the particle size reducing operation reduces the size of the particles of amorphous atorvastatin to have a particle size (d₉₀) that is less than 150 µm.

66. The method of claim 63, wherein the particle size reducing operation reduces the size of the particles of amorphous atorvastatin to have a mean particle size (d₅₀) that is between approximately 5 and 50 µm.

67. The method of claim 55, wherein the dosage form comprises between approximately 1% and 50% by weight of amorphous atorvastatin.

68. The method of claim 55, wherein the amorphous atorvastatin comprises one or more of atorvastatin calcium, atorvastatin magnesium, atorvastatin aluminum, atorvastatin iron, and atorvastatin zinc.

69. The method of claim 55, further comprising mixing the amorphous atorvastatin and alkali metal salt additive with one or more pharmaceutical excipients selected from the group comprising diluents, surfactants, antioxidants, disintegrants, binders, lubricants, glidants, and chelating agents.

70. The method of claim 55, wherein the dosage form comprises a tablet.

71. The method of claim 70, further comprising coating the tablet.

72. The method of claim 55, wherein the dosage form comprises a capsule.

73. The method of claim 55, wherein the dosage form comprises one or more of a 10 mg atorvastatin dosage unit, a 20 mg atorvastatin dosage unit, a 40 mg atorvastatin dosage unit, and an 80 mg atorvastatin dosage unit.

74. A method of treatment for a medical condition, the method comprising:
providing an oral pharmaceutical dosage comprising a therapeutically effective dosage of atorvastatin to treat the medical condition,
wherein a majority of the atorvastatin in the pharmaceutical dosage comprises amorphous atorvastatin.

75. The method of treatment of claim 74, wherein the medical condition comprises one or more of primary hypercholesterolemia, dysbetalipoproteinemia, and homozygous familial hypercholesterolemia.

76. The method of treatment of claim 74, wherein the atorvastatin of the oral pharmaceutical dosage is stabilized in the dosage by an alkali metal salt additive.

77. The method of treatment of claim 76, wherein the alkali metal salt additive is present at between approximately 1.2% and less than 5% by weight of the formulation.

78. The method of treatment of claim 74, wherein the amorphous atorvastatin comprises particles having a particle size (d₉₀) that is less than 150 µm.

79. The method of treatment of claim 74, wherein the amorphous atorvastatin comprises particles having a mean particle size (d₅₀) that is between approximately 5 and 50 µm.

80. The method of treatment of claim 74, wherein the oral pharmaceutical dosage comprises one or more of a 10 mg atorvastatin dosage unit, a 20 mg atorvastatin dosage unit, a 40 mg atorvastatin dosage unit, and an 80 mg atorvastatin dosage unit.

81. A method of stabilizing a pharmaceutical formulation comprising amorphous atorvastatin, the method comprising mixing amorphous atorvastatin with an alkali metal salt additive, the alkali metal salt additive being present at between approximately 1.2% and less than 5% by weight of the formulation.

82. A method of improving bioavailability of a pharmaceutical formulation comprising atorvastatin in comparison to a pharmaceutical formulation comprising atorvastatin marketed under the trade name Lipitor™, the method comprising reducing the particle size of the amorphous atorvastatin, wherein the particles having a particle size (d₉₀) that is less than 150 µm and a mean particle size (d₅₀) that is between approximately 5 and 50 µm.

83. A method of processing amorphous atorvastatin to reduce the particle size of the amorphous atorvastatin, the method comprising milling the amorphous atorvastatin particles using one or more of an air jet milling, ball milling, cad milling, and multi milling operation to reduce the particle size of the amorphous atorvastatin such that the particles have a particle size (d₉₀) that is less than 150 µm and a mean particle size (d₅₀) that is between approximately 5 and 50 µm.

84. A pharmaceutical formulation comprising crystalline atorvastatin and an alkali metal salt additive, the alkali metal salt additive being present at between approximately 1.2% and less than 5% by weight of the formulation.

85. The pharmaceutical formulation of claim 84, wherein the alkali metal salt additive is present at between approximately 2.0% and 4.8% by weight of the formulation.

86. The pharmaceutical formulation of claim 84, wherein the alkali metal salt is present at between approximately 4.3% and 4.4% by weight of the formulation.

87. The pharmaceutical formulation of claim 84, wherein the alkali metal salt additive comprises one or more of sodium carbonate, sodium bicarbonate, sodium hydroxide, sodium silicate, disodium hydrogen orthophosphate, sodium dihydrogen phosphate, disodium hydrogen phosphate, sodium phosphate, sodium aluminate, calcium carbonate, calcium hydroxide, magnesium carbonate, magnesium hydroxide, magnesium silicate, magnesium aluminate, and aluminum magnesium hydroxide.
